(19) **Europäisches Patentamt**
**European Patent Office**
**Office européen des brevets**

(11) **EP 4 559 398 A1**

(12) **EUROPEAN PATENT APPLICATION**

(43) Date of publication:
**28.05.2025 Bulletin 2025/22**

(51) International Patent Classification (IPC):
**A61B 6/00** (2024.01)　　**A61B 6/40** (2024.01)
**A61B 6/42** (2024.01)

(21) Application number: **23212082.4**

(22) Date of filing: **24.11.2023**

(52) Cooperative Patent Classification (CPC):
**A61B 6/405; A61B 6/4233; A61B 6/482**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Teledyne Dalsa B.V.**
**5656 AE Eindhoven (NL)**

(72) Inventor: **KORTHOUT, Alouisius Wilhelmus Marinus**
**5656 AE Eindhoven (NL)**

(74) Representative: **Arnold & Siedsma**
**Bezuidenhoutseweg 57**
**2594 AC The Hague (NL)**

(54) **X-RAY SYSTEM FOR GENERATING MULTI-SPECTRUM IMAGE DATA**

(57)　Aspects of the present disclosure relate to an x-ray system for generating multi-spectrum image data. Further aspects of the present disclosure relate to a corresponding method and to an X-ray detector that can be used in such a system.

According to an aspect of the present disclosure, an X-ray system is provided that comprises an X-ray source configured to be sequentially operable in *n* spectral modes for the purpose of sequentially emitting X-rays with a different spectrum. The X-ray system further comprises an X-ray detector that includes a pixel controller and a plurality of active pixels, and an image processing unit.

The plurality of active pixels comprises *n* pixel groups of active pixels. The pixel controller is configured to cause each pixel group to record signals that correspond to one or more of said spectral modes in dependence of said at least one synchronization signal such that at least one pixel group records signals that correspond to a plurality of said spectral modes.

The image processing unit is configured to generate multi-spectrum image data based on the signals recorded by each pixel group.

FIG. 7

**Description**

FIELD

Aspects of the present disclosure relate to an x-ray system for generating multi-spectrum image data. Further aspects of the present disclosure relate to a corresponding method and to an X-ray detector that can be used in such a system. Aspects of the present disclosure particularly relate to X-ray applications such as mammography, fluoroscopy, surgery and tomography, extra oral dental, intra oral dental, and non-medical non-destructive testing applications like e.g., pipe-line inspection and security.

BACKGROUND

X-ray systems are known in the art. These systems comprise an X-ray source for generating and emitting X-rays, an X-ray detector for detecting the X-rays, and an image processing unit for generating image data such as an X-ray image based on the detected X-rays.

Typically, the X-rays that are emitted by the X-ray source have a single spectrum that is substantially constant in time. It is known that objects or materials may react differently based on the spectrum of the X-rays. For example, some objects or materials may display a higher absorption for certain X-ray spectra than for other X-ray spectra.

In the prior art, approaches have been described in which X-rays with different spectra are used for X-ray imaging. One of the methods to acquire multi-spectrum image data is to work with a multi-spectrum source. This source can generate two or more different X-ray spectra. One X-ray spectrum can mainly have low-energy X-rays and the other X-ray spectrum can have more high-energy X-ray photons.

In prior art systems with a multi-spectrum source, two or more images are taken to get a multi-spectrum X-ray image. These two or more images are taken in succession. Out of necessity, there will be some time delay between the two images. This is a problem because the object that is being imaged can move in between the times at which the two images are taken, leading to motion artefacts. This is a problem for instance when imaging a beating heart, or when imaging the flow of blood with a contrast agent through a system of arteries in a human body.

The smaller the time delay at which the images are taken the better the quality of the multi-spectrum X-ray image. Novel X-ray sources, e.g., those based on carbon nanotubes, can generate different X-ray spectra in quick succession. It is difficult however to read the images from an image sensor very quickly. The readout of an X-ray image sensor is typically done row-by-row and the sequential readout takes some time. In many cases the X-ray detector will be limiting the minimum time delay between which two images can be taken.

SUMMARY

Aspects of the present disclosure relate to an X-system that can generate multi-spectrum image data in which the abovementioned problem does not occur or at least to a lesser extent.

According to an aspect of the present disclosure an X-ray system is provided that comprises an X-ray source configured to be sequentially operable in $n$ spectral modes in dependence of at least one synchronization signal for the purpose of sequentially emitting X-rays with a different spectrum. For example, the X-ray source may, when operating in a first spectral mode, emit X-rays having a first spectrum in a time period between t0 and t1 and, when operating in a second spectral mode, emit X-rays having a second spectrum in a time period between t2 and t3. A temporal difference between t1 and t2 can be very small, for example smaller than 1 ms or even smaller than 100us. The X-ray source can be based on carbon nanotubes, although other X-ray sources that can quickly switch between different spectra of the emitted X-rays are not excluded.

The X-ray system further includes an image processing unit and an X-ray detector that includes a pixel controller and a plurality of active pixels. Here, the pixel controller may be configured to control the active pixels by providing these pixels with appropriate control signals.

The plurality of active pixels comprises $n$ pixel groups of active pixels. For example, the plurality of pixels may comprise a matrix of 1000 x 1000 pixels that is divided into four groups of each 250,000 pixels. The pixel controller is configured to cause each pixel group to record signals that correspond to one or more of said spectral modes in dependence of said at least one synchronization signal such that at least one pixel group records signals that correspond to a plurality of said spectral modes. For example, when using four pixel groups and when the X-rays emitted by the X-ray source have, in succession, a first spectrum (Sp1), a second spectrum (Sp2), a third spectrum (Sp3), and a fourth spectrum (Sp4), the pixel groups G1-G4 may record signals in accordance with the table below:

| Pixel group | Records signals | Record signals associated with X-rays having the spectrum |
|---|---|---|
| G1 | S1+S2+S3+S4 | Sp1+Sp2+Sp3+Sp4 |
| G2 | S2+S3+S4 | Sp2+Sp3+Sp4 |
| G3 | S3+S4 | Sp3+Sp4 |
| G4 | S4 | Sp4 |

The image processing unit is configured to generate multi-spectrum image data based on the signals recorded by each pixel group. In some X-ray detectors, for example those based on 3T, 4T or 5T pixel topologies, incoming X-rays are converted by a scintillator into visible light photons. These photons are then absorbed by a photoconductor or other light sensitive element in the pixel that generates or releases charged particles, such as electrons. These charged particles can be collected and stored in a storage capacitor that is arranged within each pixel. The voltage generated by the charged particles can be read out. In this particular embodiment, the signals recorded by the pixels in a given pixel group may include or may be related to the voltages over the corresponding storage capacitors.

The image processing unit can be configured to determine, for each pixel, as a primary signal that is associated with the pixel group to which said pixel belongs, the signal recorded by that pixel. It may further be configured to compute, for that pixel and for each of the remaining pixel groups to which that pixel does not belong, a respective secondary signal by combining signals recorded by neighboring pixels that belong to a same pixel group. The image processing unit can be configured to determine the multi-spectrum image data for that pixel based on the primary signal and the respective secondary signal(s).

The X-ray source, when operating in an $i$-th spectral mode among the $n$ spectral modes, can be configured to emit X-rays having an $i$-th spectrum. Each spectrum among the $n$ spectra can be different. Additionally, or alternatively, the pixel controller can be configured to cause each pixel group among the $n$ pixel groups to record signals that correspond to a different segment of the sequence of X-rays with different spectra. For example, the sequence of X-rays may correspond to X-rays with spectra Sp1, Sp2, Sp3, and Sp4 that were emitted sequentially. The pixels in different pixel groups may record different segments of this sequence. An example of a segment of a sequence can be {Sp2, Sp3, Sp4} or {Sp3, Sp4}. It is noted that each segment corresponds to the X-rays that were sequentially emitted. For example, {Sp1, Sp3} is not a segment.

Each different segment of the sequence of X-rays with different spectra may have initial X-rays and final X-rays, wherein a spectrum of the final X-rays for each segment can be the same. For example, if the sequence {Sp1, Sp2, Sp3, Sp4} is emitted by the X-ray source, a given pixel group may record a segment of this sequence, for example {Sp3, Sp4}. For this segment of the sequence, the initial X-rays correspond to spectrum Sp3, whereas the final X-rays correspond to spectrum Sp4. Stating that the final X-rays for each segment are the same would for this example result in the following different segments that can be recorded by a pixel group: {Sp1, Sp2, Sp3, Sp4}, i.e. the segment corresponds to the entire sequence, { Sp2, Sp3, Sp4}, { Sp3, Sp4}, and {Sp4}.

A temporal length of the segment of the sequence of X-rays may correspond to a cumulated time period in which the X-ray source operates in the corresponding spectral modes. In the example above, the temporal length of the segment recorded by the pixels of a given pixel group corresponds to the time during which the X-ray source operated in the corresponding spectral modes. Alternatively, a pixel in a pixel group may be configured to record the X-rays for a given spectrum only during a part of the corresponding spectral mode.

The pixels of the plurality of pixels can be arranged in a matrix of rows and columns, wherein the matrix of rows and columns comprise a regular repetition of a unit cell, wherein the unit cell comprises at least one pixel of each pixel group. At least some pixels of the same pixel group but belonging to a different unit cell can be adjacently arranged. In this case, each row or each column of pixels may only comprise pixels that belong to the same pixel group. Alternatively, each pair of pixels of the same pixel group but belonging to a different unit cell may not be adjacently arranged.

Each active pixel can be a 3T, 4T, or 5T pixel, preferably implemented using complementary metal oxide semiconductor, CMOS, technology. Furthermore, each active pixel may comprise a storage capacitor and a photodetector having a photosensitive area. The photodetector typically comprises a photodiode. It should be noted that 3T, 4T, and 5T pixel topologies are well known in the art.

The present disclosure equally relates to indirect X-ray detectors, in which a scintillator layer is disposed over the pixels for converting the X-ray photons into visible light photons, and to direct X-ray detectors. In these latter detectors, the scintillator layer is replaced by a direct conversion material, such as Cadmium Zinc Telluride or Cadmium Telluride.

For each pixel group, the active pixels of that pixel group can be identical. Furthermore, the active pixels belonging to different pixel groups may have a different size of the photosensitive area. For a pair of pixel groups among the $n$ pixel groups of which the active pixels have a different size of the photosensitive area, active pixels belonging to the pixel group of which the pixels have a larger photosensitive area can be configured to record a smaller part of the sequence of X-rays with different spectra than active pixels belonging to the pixel group of which the pixels have a smaller photosensitive area. In this manner, the storage capacitor can be substantially identical for all pixels. More specifically, the smaller photosensitive area is at least partially compensated by the fact that these pixels record a larger part of the sequence of X-rays.

Alternatively, for a pair of pixel groups among the $n$ pixel groups of which the active pixels have a different size of the photosensitive area, active pixels belonging to the pixel group of which the pixels have a larger photosensitive area can have a larger storage capacitor and can be configured to record a larger part of the sequence of X-rays with different spectra when compared to active pixels belonging to the pixel group of which the pixels have a smaller photosensitive area. In this approach, some of the pixel groups, i.e., those having the larger photosensitive area, may be used to mainly record or determine the overall intensity of the X-rays, whereas the other pixel groups may be used to determine the spectral distribution.

Alternatively, for a pair of pixel groups among the $n$ pixel groups of which the active pixels have an identical size of the photosensitive area but for which the capacitance of the storage capacitor is different, active pixels belonging to the pixel group of which the pixels have a larger storage capacitor can be configured to record a larger part of the sequence of X-rays with different spectra when compared to active pixels belonging to the pixel group of which the pixels have a smaller storage capacitor.

It should be noted that the present disclosure equally relates to using pixel groups wherein pixels from different pixel groups differ both in size of the photosensitive area and in capacitance of the storage capacitor.

The pixel controller can be configured to provide a separate select signal for each row of pixels, wherein pixels arranged in a same column are connected to a same column line. Furthermore, the X-ray detector may comprise a readout unit that includes a separate readout circuit for each column line. The readout circuit may be configured to read the signal from a selected pixel on a column line. In some embodiments, the readout circuit uses a correlated double sampling, CDS, or differential double sampling, DDS, technique for obtaining the signal from the selected pixel. In addition, the pixel controller can be configured to provide different reset signals for pixels that are arranged in the same row but belong to different pixel groups.

The pixel controller can be configured to simultaneously stop resetting all pixels in a pixel group for allowing these pixels to record signals corresponding to the sequence of X-rays with different spectra or to a part thereof. Typically, when resetting the pixel is stopped, the pixel will start recording a signal, e.g., the photodiode will output charged particles such as electrons that are stored in the storage capacitor.

The image processing unit can be integrated inside the X-ray detector or the image processing unit can be arranged separate from the X-ray detector.

The at least one synchronization signal can be generated by the X-ray detector. In this case, the X-ray source is configured to start sequentially emitting X-rays with a different spectrum in dependence of said at least one synchronization signal. Alternatively, the at least one synchronization signal can be generated by the X-ray source. In this case, the X-ray detector is configured to start recording the signals that correspond to the spectral modes in dependence of said at least one synchronization signal. Alternatively, the at least one synchronization signal can be generated by a controller. In this case, the X-ray source can be configured to start sequentially emitting X-rays with a different spectrum in dependence of said at least one synchronization signal, and the X-ray detector can be configured to start recording the signals that correspond to the spectral modes in dependence of said at least one synchronization signal.

The at least one synchronization signal may comprise a respective synchronization signal for each of the $n$ spectral modes. More specifically, a synchronization signal will be provided or generated each time X-rays with a new spectrum are being emitted. Alternatively, a single synchronization signal is used to mark the beginning of a sequence of X-rays with different spectra. In this embodiment, each spectral mode has a known time duration and temporal position relative to the start of the sequence. This information should be known to both the X-ray source and to the X-ray detector.

According to a second aspect of the present disclosure, an X-ray detector is provided as described above.

According to a third aspect of the present disclosure, a method for obtaining multi-spectrum X-ray image data is provided that comprises the steps of sequentially operating an X-ray source in $n$ spectral modes in dependence of at least one synchronization signal for sequentially emitting X-rays with a different spectrum, using $n$ pixel groups of active pixels of an X-ray detector to each record signals that correspond to one or more of said spectral modes in dependence of said at least one synchronization signal such that at least one pixel group records signals that correspond to a plurality of said spectral modes, and generating multi-spectrum image data based on the signals recorded by each pixel group.

DESCRIPTION OF THE DRAWINGS

So that the manner in which the features of the present disclosure can be understood in detail, a more particular description is made with reference to embodiments, some of which are illustrated in the appended figures. It is to be noted, however, that the appended figures illustrate only typical embodiments and are therefore not to be considered limiting of its scope. The figures are for facilitating an understanding of the disclosure and thus are not necessarily drawn to scale.

Advantages of the subject matter claimed will become apparent to those skilled in the art upon reading this description in conjunction with the accompanying figures, in which like reference numerals have been used to designate like elements, and in which:

Figure 1 illustrates an X-ray system in accordance with the present disclosure;
Figure 2 illustrates the X-ray detector of the X-ray system of figure 1;
Figure 3 illustrates details of the X-ray detector of figure 2;
Figure 4 illustrates a pixel of the X-ray detector of figure 2;
Figure 5 illustrates a connection of reset signals for pixels of the X-ray detector of figure 2;
Figure 6 illustrates a timing diagram of the X-ray system of figure 1;
Figure 7 illustrates the computation of pixel signals using the X-ray detector of figure 2; and
Figure 8 illustrates a method in accordance with the present disclosure.

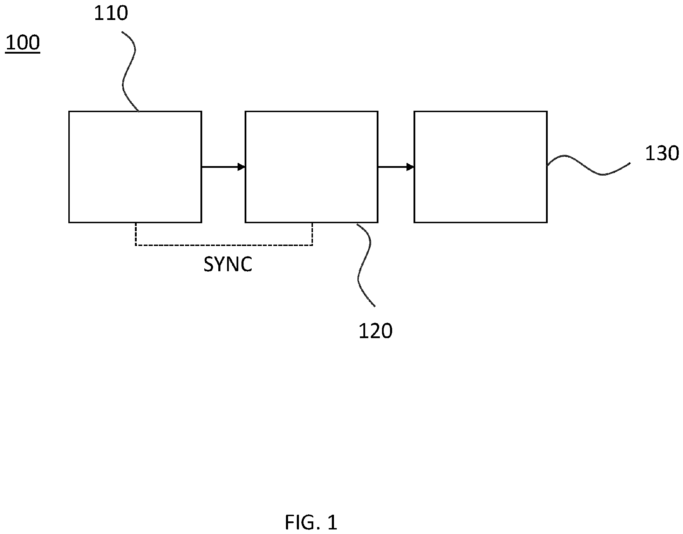

Figure 1 illustrates an X-ray system 100 in accordance with the present disclosure. It comprises an X-ray source 110 that is capable of generating X-rays with different spectra. More in particular, it is capable of generating a sequence of X-rays, comprising a plurality of bursts or pulses of X-rays, wherein at least some of the bursts or pulses have a different energy spectrum. These types of sources are known in the art and may comprise carbon nanotube-based X-ray sources.

X-ray system 100 further comprises an X-ray detector 120 for recording signals that correspond to the generated X-rays, and an image processing unit 130 to generate multi-spectrum image data based on the signals recorded by X-ray detector 120.

As indicated, X-ray system 100 and X-ray detector 120 are synchronized using at least one synchronization signal SYNC. This signal marks the moments in time when X-ray source starts to emit X-rays. In a particular embodiment, X-ray source 110 is capable of generating a sequence comprising $n$ bursts or pulse of X-rays, wherein at least some of these bursts or pulses have a different energy spectrum. Furthermore, in this embodiment, the at least one synchronization signal may comprise $n$ synchronization signals to each mark a respective burst or pulse. In other embodiments, less synchronization signals are used and in a particular embodiment, only a single synchronization signal is used to mark the first burst or pulse. In this embodiment, the X-ray source will start a predefined sequence of bursts or pulses of which the timing information, with the exception of the starting time of the sequence, is known to the X-ray detector.

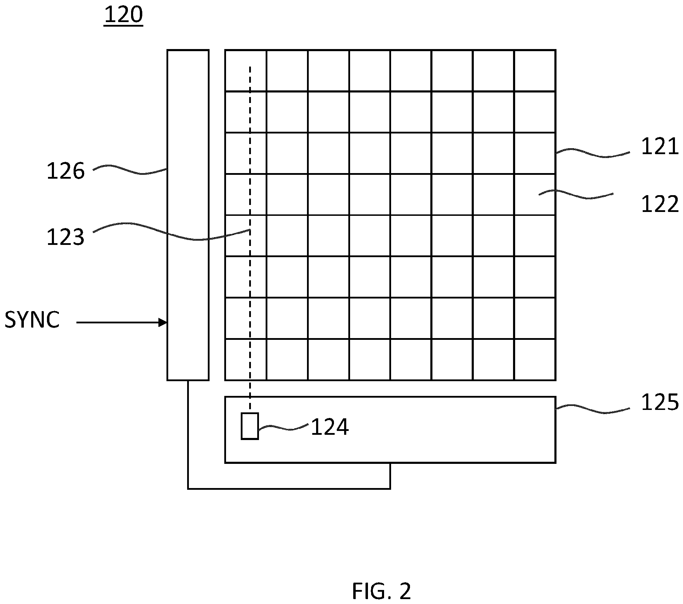

Figure 2 illustrates X-ray detector 120 in more detail. It comprises a matrix 121 of rows and columns of active pixels 122. Each pixel 122 is connected to a respective column line 123 of which one example is shown in figure 2. Each column line 123 is connected to a readout circuit 124 comprised in readout unit 125. Each readout circuit is capable of reading out a signal from a single selected pixel connected to the relevant column line 123 at a time.

X-ray detector 120 further comprises a pixel controller 126 that generates various control signals for controlling pixels 122. For example, pixel controller 126 generates reset signals for resetting pixels 122 and select signals for selecting pixels 122 for readout. Depending on pixel topology, pixel controller 126 may generate further control signals, such as transfer gate control signals for 4T and 5T pixels.

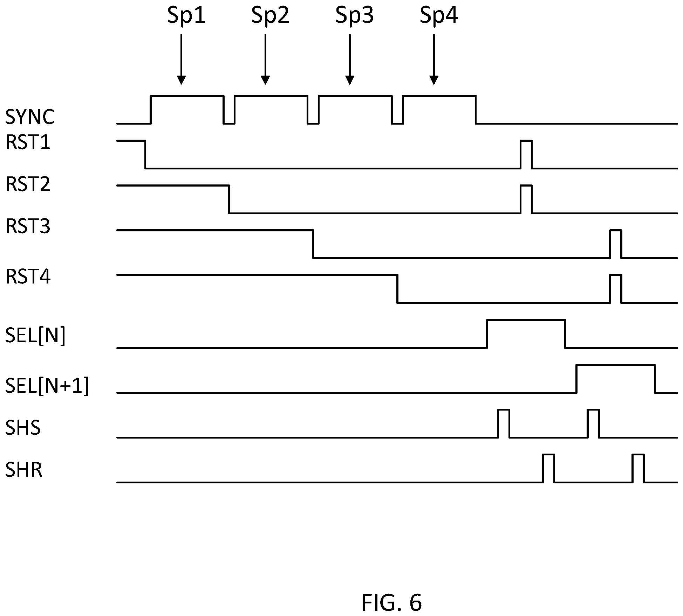

Pixel controller 126 receives or generates the at least one synchronization signal SYNC as will be described later in connection with figure 6. Pixel controller 126 further provides control signals to readout unit 125.

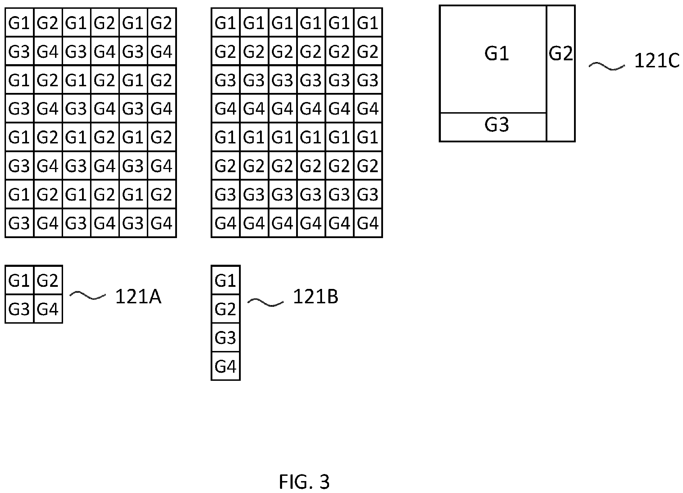

Figure 3 illustrates details of X-ray detector 120. Figure 3 illustrates as a left figure and center figure, two different configurations of pixels 122. More in particular, the plurality of pixels 122 is divided into pixel groups. For example, in the configuration shown on the left, the matrix of pixels 122 is constructed using a repetition of a unit cell 121A that comprises a single pixel belonging to a first group G1, a single pixel belonging to a second group G2, a single pixel belonging to a third group G3, and a single pixel belonging to a fourth group G4. As shown, each row of matrix 121 comprises pixels 122 of different groups.

In the configuration shown in the center, unit cell 121B also comprises a single pixel 122 for each of the four groups. However, in this case, each row of matrix 121 comprises pixels 122 of only a single group.

In the left and center configurations, pixels 122 are identical. However, the present disclosure is not limited thereto. For example, on the right, figure 3 illustrates a unit cell 121C comprising a pixel for each of three groups. As shown, the pixel for group G1 is much larger than the pixels for groups G2 and G3.

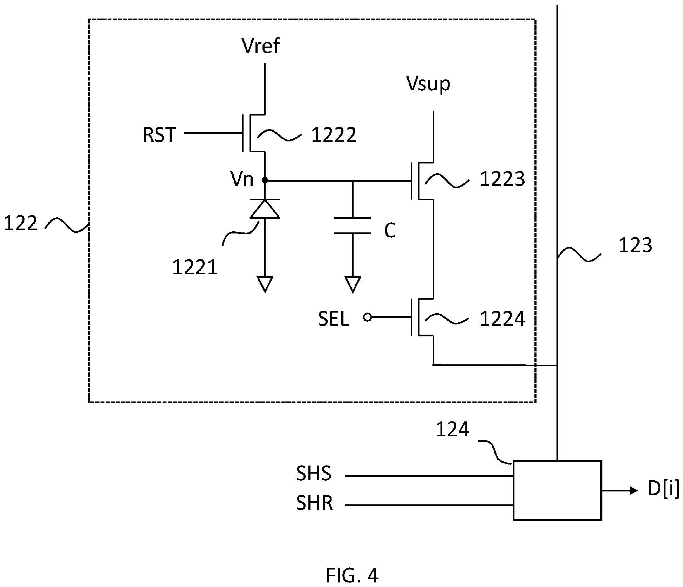

Figure 4 illustrates a pixel 122 of X-ray detector 120. Pixel 122 has a known 3T topology and comprises a photodiode 1221 that outputs electrons as a result of absorbing photons. These electrons are collected by storage capacitor C. The voltage over storage capacitor C can be reset in dependence of a reset signal RST using reset transistor 1222 that is connected to a reference voltage Vref.

The voltage over storage capacitor C can be put on column line 123 in dependence of a select signal SEL using a source follower 1223 and select transistor 1224. Signals on column line 123 are readout using readout circuitry 124.

Next, an exemplary mode of operation for pixel 122 will be described. First, pixel 122 will be in a reset condition with signal RST having a logical high value. This will force the voltage over storage capacitor, hereinafter referred to as Vn, to be equal to Vref. If the at least one synchronization signal indicates that X-ray capture should commence, signal RST will attain a logical low value. As a result, the current outputted by photodiode 1221 will start to discharge storage capacitor C. After a predetermined amount of time, voltage Vn will be read out. To this end, signal SEL and signal SHS are set to a logical high value. This will cause voltage Vn minus the threshold voltage Vth of source follower 1223 to be put on column line 123 and to be stored within readout circuit 124. It is noted that voltage Vn at this time equals Vref-Vphoto, wherein Vphoto is the magnitude of the voltage drop associated with the electrons generated by photodiode 1221.

Next, signal SHS will be set to a logical low value and signal RST will be set to a logical high value. This will cause voltage Vn to be reset to Vref. After this reset, signal SHR will be set to a logical high and voltage Vn=Vref minus threshold voltage Vth will be put on column line 123 and stored within readout circuit 124.

At this stage, readout circuit 124 constructs a pixel voltage using the previously stored values. By subtracting these values, i.e., (Vref-Vth)-(Vref-Vphoto-Vref)=Vphoto, information can be obtained about the amount of photons converted by pixel 122. Vphoto can subsequently be transformed into the digital domain to obtain image data for pixel 122.

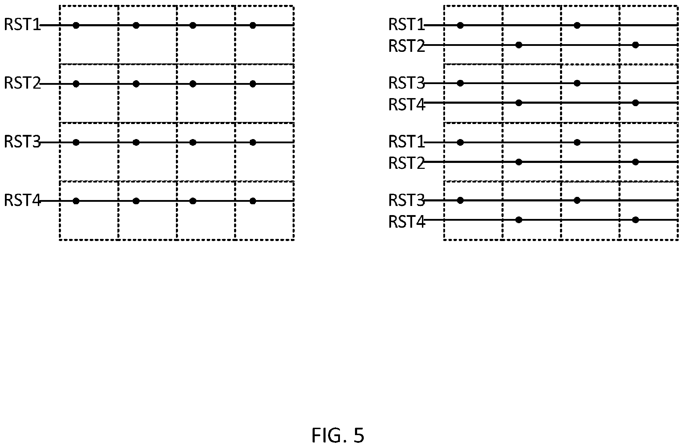

Figure 5 illustrates a connection of reset signals for pixels 122 of X-ray detector 120. The configuration shown on the left corresponds to the center configuration of figure 3. More in particular, each row comprises pixels 122 of a single pixel group and the corresponding RST signal is distributed to each and every pixel 122 in that row.

The configuration shown on the right corresponds to the configuration on the left in figure 3 wherein each row comprises pixels of two different groups. Therefore, for each row, two RST signals need to be distributed. As an example, for a 1000 x 1000 pixel matrix, the configuration shown on the left in figure 5 would require 1000 RST signals to be generated by pixel controller 126, whereas the configuration shown on the right in figure 5 would require 2000 RST signals to be generated by pixel controller 126.

Figure 6 illustrates a timing diagram of the X-ray system of figure 1. Figure 6 illustrates an example in which four pixels groups are used, and in which X-ray source emits a sequence of X-rays with energy spectra Sp1, Sp2, Sp3, Sp4. Generating these X-rays is controlled and/or described by the SYNC signal.

Prior to the emission of the X-rays, reset signals RST1-RST4 for the four groups are all at a logical high level. Just before X-rays with the first spectrum Sp1 are generated and emitted, the reset signal for the first pixel group, i.e., RST1, will attain a logical low value causing the pixels in that pixel group to process the signals for first spectrum Sp1. Just before X-rays with second spectrum Sp2 are generated and emitted, the reset signal for the second pixel group, i.e., RST2, will attain a logical low value causing the pixels in that pixel group and those in the first pixel group to process the signals for second spectrum Sp2. Just before X-rays with third spectrum Sp3 are generated and emitted, the reset signal for the third pixel group, i.e., RST3, will attain a logical low value causing the pixels in that pixel group and those in the first and second pixel groups to process the signals for third spectrum Sp3. And finally, just before X-rays with fourth spectrum Sp4 are generated and emitted, the reset signal for the fourth pixel group, i.e., RST4, will attain a logical low value causing the pixels in that pixel group and those in the first, second, and third pixel groups to process the signals for fourth spectrum Sp4.

After the X-rays with the fourth spectrum Sp4 have been emitted, the signals stored in matrix 121 will be read out on a row-by-row basis. This process is illustrated for rows N and N+1. It should be noted that the timing diagram of figure 6 corresponds to the configuration shown on the left in figure 3, i.e., with pixels from two different pixel groups arranged per row.

To read out row N, the corresponding select signal SEL[N] and signal SHS will be set to a logical high to allow the voltage over the storage capacitor to be read out and stored in the readout circuitry. This process will be performed for all columns simultaneously. Next, reset signals RST1 and RST2 will be set to a logical high value for resetting the pixels of groups 1 and 2 in row N. Directly after reset, signal SHR will be set to a logical high value to allow the reference voltage to be read out as described in connection with figure 4. This step is also performed for all the pixels in groups 1 and 2 in row N. After this step, signal SEL[N] will be set to a logical low value and the values for Vphoto will be transferred as image data to a memory in readout unit 125 for all pixels in row N.

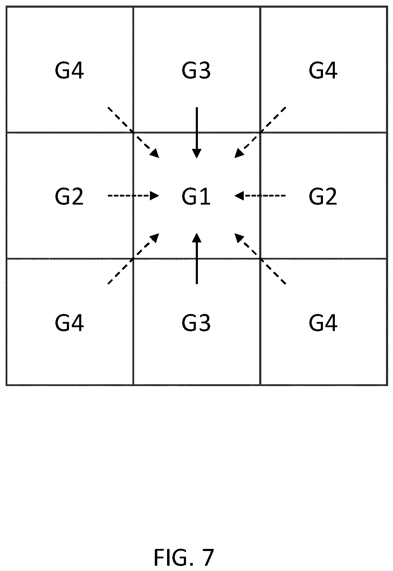

Next, signal SEL[N+1] will be set to a logical high value and the abovementioned process will be repeated for row N+1. Once all rows have been processed, readout unit 125 has image data for each pixel 122 in matrix 121. These data will be transferred to image processing unit 130 for further processing, which will be described next referring to figure 7.

As can be derived from the above, the reset signals are provided to the pixels differently depending on the fact if any signals are stored in pixel matrix 121 or not. For example, the reset signals are provided to all pixels simultaneously prior to the emission of the X-rays. Such reset is called a global reset. However, when reading out the signals on a row-by-row basis, the reset signals are also set on a row-by-row basis to avoid resetting pixels that have not yet been read out. It should be noted that if the process is to be repeated for a next sequence of X-rays with different spectra, it may not be required to use a global reset prior to acquiring the signals as the pixels have already been reset during pixel readout.

As described above, for each pixel image data is available. However, these data may refer to different segments of the sequence of X-rays. For example, pixels belonging to pixel group 1 have processed X-rays belonging to spectra Sp1-Sp4, pixels belonging to pixel group 2 have processed X-rays belonging to spectra Sp2-Sp4, pixels belonging to pixel group 3 have processed X-rays belonging to spectra Sp3-Sp4, and pixels belonging to pixel group 4 have processed X-rays belonging to spectra Sp4.

If the pixels on the pixel matrix would each receive the same amount of X-rays per energy spectrum, provided that these pixels are configured to process these spectra, the energy spectra could be decomposed quite easily. For example, a pixel belong to group 1 would have received signals S1+S2+S3+S4 corresponding to spectra Sp1, Sp2, Sp3, and Sp4, respectively, and a pixel belonging to group 2 would have received signals S2+S3+S4 corresponding to spectra Sp2, Sp3, Sp4, respectively. The signal associated with spectrum Sp1 could then be determined by simply subtracting the signals associated with the pixel belonging to group 2 from the signals of the pixel belonging to group 1 to provide signal S1.

The abovementioned approach does not work for actual images because pixels in the same pixel group will generate different signals, and because the signals associated with a given spectrum differ between pixels of different groups due to the fact that these pixels are not on the same position in the pixel matrix.

Figure 7 illustrates an approach to address this problem. In this approach, to compute the energy spectra for the center pixel, which belongs to group G1, hypothetical secondary signals will be computed using signals from neighboring pixels of other pixel groups.

The signal obtained by pixel G1 itself will be called the primary signal PRIM and corresponds to S1(P1)+S2(P1)+S3(P1)+S4(P1), wherein Sx(Py) refers to the signal corresponding to spectrum *x* as received by a pixel from pixel group *y*. As shown in figure 7, signals from neighboring pixels that belong to the same pixel group are used. To distinguish between these pixels, they will be referred to using a matrix notation. For example, P4(1,1) refers to a pixel of group G4 in the upper left corner in figure 7, P4(3,1) refers to a pixel of group G4 in the lower left corner in figure 7, P4(1,3) refers to a pixel of group G4 in the upper right corner in figure 7, and P4(3,3)) refers to a pixel of group G4 in the lower right corner in figure 7. Using this notation, the following signals can be constructed for pixel P1(2,2):

$$\text{Primary signal PRIM} = S1(P1(2,2)) + S2(P1(2,2)) + S3(P1(2,2)) + S4(P1(2,2))$$

$$\text{First secondary signal SEC1} =$$
$$(S2(P2(2,1)) + S2(P2(2,3)))/2 + (S3(P2(2,1)) + S3(P2(2,3)))/2 + (S4(P2(2,1)) + S4(P2(2,3)))/2$$

$$\text{Second secondary signal SEC2} =$$
$$(S3(P3(1,2)) + S3(P3(3,2)))/2 + (S4(P3(1,2)) + S4(P3(3,2)))/2$$

$$\text{Third secondary signal SEC3} =$$
$$(S4(P4(1,1)) + S4(P4(1,3)) + S4(P4(3,1)) + S4(P4(3,3)))/4$$

First secondary signal SEC1 is an estimation of the signal received by pixel P1(2,2) had this pixel only processed X-rays with energy spectra S2, S3, S4. Using the primary signal and the secondary signals SEC1, SEC2, SEC3, the energy spectra corresponding to pixel P1(2,2) can be computed using:

$$S1(P1(2,2)) = PRIM - SEC1$$

$$S2(P1(2,2)) = PRIM - S1(P1(2,2)) - SEC2$$

$$S3(P1(2,2)) = PRIM - S1(P1(2,2)) - S2(P1(2,2)) - SEC3$$

$$S4(P1(2,2)) = PRIM - S1(P1(2,2)) - S2(P1(2,2)) - S3(P1(2,2)) - SEC4$$

wherein the calculations for S2, S3, and S4 use data calculated for a previous spectrum.

It should be apparent to the skilled person that the calculation described above would change if a different configuration of the pixels in pixel matrix 121 were used. Furthermore, the present disclosure is not limited to using identical pixels in pixel matrix 121. This has already been demonstrated in figure 3. In case pixels with different areas of the photosensitive area and/or different capacitances of the storage capacitor are used, a scaling would need to be introduced in the method described above. For example, assuming the size of the photosensitive area of the pixels in pixel group G1 is *k* times larger than the size of the photosensitive area of the pixels in pixel group G4, and if the capacitance of the storage capacitor of the pixels in pixel group G1 is m times larger than the capacitance of the storage capacitor of the pixels in pixel group G4, third secondary signal SEC3 would be calculated using *k / m* $\times$ ((S4(P4(1,1))+S4(P2(1,3))+ S4(P4(3, 1))+S4(P4(3,3)))/4).

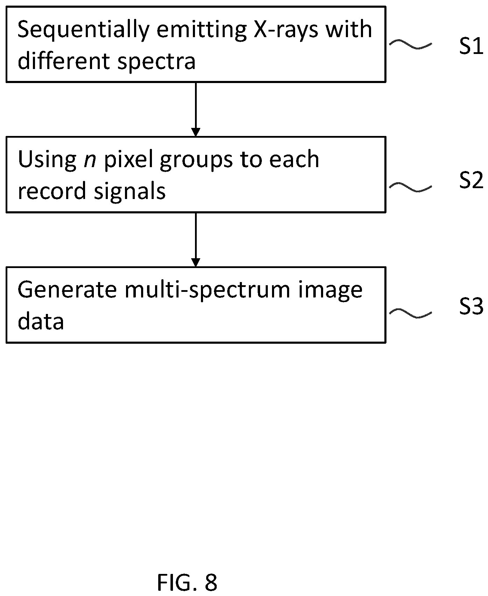

Figure 8 illustrates a method for obtaining multi-spectrum X-ray image data. It comprises a step S1 of sequentially operating an X-ray source in $n$ spectral modes in dependence of at least one synchronization signal for sequentially emitting X-rays with a different spectrum, a step S2 of using $n$ pixel groups of active pixels of an X-ray detector to each record signals that correspond to one or more of said spectral modes in dependence of said at least one synchronization signal such that at least one pixel group records signals that correspond to a plurality of said spectral modes, and a step S3 of generating multi-spectrum image data based on the signals recorded by each pixel group.

In the above, the present invention has been described using detailed embodiments thereof. However, the present invention is not limited to these embodiments. Instead, various modifications are possible without departing from the scope of the present invention which is defined by the appended claims and their equivalents.

For example, aspects of the present disclosure are related to X-ray systems using active pixels. However, the present disclosure is not limited thereto. For example, the pixels of the X-ray detector may equally be passive pixels.

Particular and preferred aspects of the invention are set out in the accompanying independent claims. Combinations of features from the dependent and/or independent claims may be combined as appropriate and not merely as set out in the claims.

The scope of the present disclosure includes any novel feature or combination of features disclosed therein either explicitly or implicitly or any generalization thereof irrespective of whether or not it relates to the claimed invention or mitigate against any or all of the problems addressed by the present invention. The applicant hereby gives notice that new claims may be formulated to such features during prosecution of this application or of any such further application derived therefrom. In particular, with reference to the appended claims, features from dependent claims may be combined with those of the independent claims and features from respective independent claims may be combined in any appropriate manner and not merely in specific combinations enumerated in the claims.

Features which are described in the context of separate embodiments may also be provided in combination in a single embodiment. Conversely, various features which are, for brevity, described in the context of a single embodiment, may also be provided separately or in any suitable sub combination.

The term "comprising" does not exclude other elements or steps, the term "a" or "an" does not exclude a plurality. Reference signs in the claims shall not be construed as limiting the scope of the claims.

## Claims

1. An X-ray system (100), comprising:

   an X-ray source (110) configured to be sequentially operable in $n$ spectral modes in dependence of at least one synchronization signal (SYNC) for the purpose of sequentially emitting X-rays with a different spectrum (Sp1, Sp2, Sp3, Sp4);
   an X-ray detector (120) comprising a pixel controller (126) and a plurality of active pixels (122); and
   an image processing unit (130);
   wherein the plurality of active pixels comprises $n$ pixel groups (G1, G2, G3, G4) of active pixels;
   wherein the pixel controller is configured to cause each pixel group to record signals that correspond to one or more of said spectral modes in dependence of said at least one synchronization signal such that at least one pixel group records signals that correspond to a plurality of said spectral modes; and
   wherein the image processing unit is configured to generate multi-spectrum image data based on the signals recorded by each pixel group.

2. The X-ray system according to claim 1, wherein the image processing unit is configured to determine, for each pixel:

   determine as a primary signal that is associated with the pixel group to which said pixel belongs, the signal recorded by that pixel;
   for each of the remaining pixel groups to which said pixel does not belong, compute a respective secondary signal by combining signals recorded by neighboring pixels that belong to a same pixel group;
   wherein the image processing unit is configured to determine the multi-spectrum image data for said pixel based on the primary signal and the respective secondary signal(s).

3. The X-ray system according to claim 1 or 2, wherein the X-ray source, when operating in an i-th spectral mode among the $n$ spectral modes, emits X-rays having an i-th spectrum, wherein each spectrum among the $n$ spectra is preferably different.

4. The X-ray system according to any of the previous claims, wherein the pixel controller is configured to cause each pixel group among the *n* pixel groups to record signals that correspond to a different segment of the sequence of X-rays with different spectra;

wherein each different segment of the sequence of X-rays with different spectra preferably has initial X-rays and final X-rays, wherein the final X-rays for each segment are the same; and
wherein, more preferably, a temporal length of the segment of the sequence of X-rays corresponds to a cumulated time period in which the X-ray source operates in the corresponding spectral modes.

5. The X-ray system according to any of the previous claims, wherein the pixels of the plurality of pixels are arranged in a matrix (121) of rows and columns, wherein the matrix of rows and columns comprise a regular repetition of a unit cell (121A, 121B, 121C), the unit cell comprising at least one pixel of each pixel group;
wherein, preferably:

at least some pixels of the same pixel group but belonging to a different unit cell are adjacently arranged, wherein each row or each column of pixels preferably only comprises pixels that belong to the same pixel group; or
each pair of pixels of the same pixel group but belonging to a different unit cell are not adjacently arranged.

6. The X-ray system according to claim 5, wherein each active pixel is a 3T, 4T, or 5T pixel, preferably implemented using complementary metal oxide semiconductor, CMOS, technology, and wherein each active pixel comprises a storage capacitor (C) and a photodetector (1221) having a photosensitive area.

7. The X-ray system according to claim 6, wherein, for each pixel group, the active pixels of that pixel group are identical, and wherein the active pixels belonging to different pixel groups have a different area of the photosensitive area.

8. The X-ray system according to claim 7, wherein, for a pair of pixel groups among the *n* pixel groups of which the active pixels have a different size of the photosensitive area:
active pixels belonging to the pixel group of which the pixels have a larger photosensitive area are configured to record a smaller part of the sequence of X-rays with different spectra than active pixels belonging to the pixel group of which the pixels have a smaller photosensitive area.

9. The X-ray system according to claim 7, wherein, for a pair of pixel groups among the *n* pixel groups of which the active pixels have a different size of the photosensitive area:
active pixels belonging to the pixel group of which the pixels have a larger photosensitive area have a larger storage capacitor and are configured to record a larger part of the sequence of X-rays with different spectra when compared to active pixels belonging to the pixel group of which the pixels have a smaller photosensitive area.

10. The X-ray system according to any of the claims 6-9, wherein the pixel controller is configured to provide a separate select signal (SEL) for each row of pixels, wherein pixels arranged in a same column are connected to a same column line (123);

wherein the X-ray detector comprises a readout unit (125) that includes a separate readout circuit (124) for each column line;
wherein the pixel controller is configured to provide different reset signals for pixels that are arranged in the same row but belong to different pixel groups.

11. The X-ray system according to any of the claims 6-10, wherein the pixel controller is configured to simultaneously stop resetting all pixels in a pixel group for allowing these pixels to record signals corresponding to the sequence of X-rays with different spectra or to a part thereof.

12. The X-ray system according to any of the previous claims, wherein the image processing unit is integrated inside the X-ray detector or wherein the image processing unit is arranged separate from the X-ray detector.

13. The X-ray system according to any of the previous claims, wherein the at least one synchronization signal:

is generated by the X-ray detector, and wherein the X-ray source is configured to start sequentially emitting X-rays with a different spectrum in dependence of said at least one synchronization signal; or
is generated by the X-ray source, and wherein the X-ray detector is configured to start recording the signals that correspond to the spectral modes in dependence of said at least one synchronization signal; or

is generated by a controller, wherein the X-ray source is configured to start sequentially emitting X-rays with a different spectrum in dependence of said at least one synchronization signal, and wherein the X-ray detector is configured to start recording the signals that correspond to the spectral modes in dependence of said at least one synchronization signal;

wherein said at least one synchronization signal preferably comprises a respective synchronization signal for each of the $n$ spectral modes.

14. An X-ray detector (120) as defined in any of the previous claims.

15. A method for obtaining multi-spectrum X-ray image data, comprising:

sequentially operating (S1) an X-ray source in $n$ spectral modes in dependence of at least one synchronization signal for sequentially emitting X-rays with a different spectrum;

using (S2) $\eta$ pixel groups of active pixels of an X-ray detector to each record signals that correspond to one or more of said spectral modes in dependence of said at least one synchronization signal such that at least one pixel group records signals that correspond to a plurality of said spectral modes; and

generating (S3) multi-spectrum image data based on the signals recorded by each pixel group.

100

110

130

120

SYNC

FIG. 1

FIG. 2

| G1 | G2 | G1 | G2 | G1 | G2 |
|----|----|----|----|----|----|
| G3 | G4 | G3 | G4 | G3 | G4 |
| G1 | G2 | G1 | G2 | G1 | G2 |
| G3 | G4 | G3 | G4 | G3 | G4 |
| G1 | G2 | G1 | G2 | G1 | G2 |
| G3 | G4 | G3 | G4 | G3 | G4 |
| G1 | G2 | G1 | G2 | G1 | G2 |
| G3 | G4 | G3 | G4 | G3 | G4 |

| G1 | G1 | G1 | G1 | G1 | G1 |
|----|----|----|----|----|----|
| G2 | G2 | G2 | G2 | G2 | G2 |
| G3 | G3 | G3 | G3 | G3 | G3 |
| G4 | G4 | G4 | G4 | G4 | G4 |
| G1 | G1 | G1 | G1 | G1 | G1 |
| G2 | G2 | G2 | G2 | G2 | G2 |
| G3 | G3 | G3 | G3 | G3 | G3 |
| G4 | G4 | G4 | G4 | G4 | G4 |

121C

| G1 | G2 |
|----|----|
| G3 | G4 |

121A

| G1 |
|----|
| G2 |
| G3 |
| G4 |

121B

FIG. 3

13

FIG. 4

FIG. 5

FIG. 6

FIG. 7

Sequentially emitting X-rays with different spectra     ∼   S1

Using $n$ pixel groups to each record signals     ∼   S2

Generate multi-spectrum image data     ∼   S3

FIG. 8

## EUROPEAN SEARCH REPORT

**Application Number**

EP 23 21 2082

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/233163 A1 (NISHIJIMA AKIRA [JP]) 27 July 2023 (2023-07-27) | 1,3-6, 10-15 | INV. A61B6/00 |
| Y | * paragraphs [0081], [0090], [0040]; figure 5 * | 2,7-9 | A61B6/40 A61B6/42 |
| Y | US 2008/135789 A1 (DU YANFENG [US] ET AL) 12 June 2008 (2008-06-12) * paragraphs [0008], [0005] * | 2 | |
| Y | US 2015/063527 A1 (DAERR HEINER [DE] ET AL) 5 March 2015 (2015-03-05) * paragraph [0044] * | 7-9 | |
| X | JP 7 199920 B2 (CANON MEDICAL SYSTEMS CORP) 6 January 2023 (2023-01-06) * paragraph [0077]; figure 3 * | 1,14,15 | |

**TECHNICAL FIELDS SEARCHED (IPC)**

A61B

The present search report has been drawn up for all claims

| Place of search | Date of completion of the search | Examiner |
|---|---|---|
| Munich | 16 April 2024 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 21 2082

16-04-2024

| Patent document cited in search report | | Publication date | Patent family member(s) | | Publication date |
|---|---|---|---|---|---|
| US 2023233163 | A1 | 27-07-2023 | CN | 116491967 A | 28-07-2023 |
| | | | JP | 2023108512 A | 04-08-2023 |
| | | | US | 2023233163 A1 | 27-07-2023 |
| US 2008135789 | A1 | 12-06-2008 | NONE | | |
| US 2015063527 | A1 | 05-03-2015 | CN | 104220899 A | 17-12-2014 |
| | | | EP | 2831630 A2 | 04-02-2015 |
| | | | EP | 3088918 A2 | 02-11-2016 |
| | | | JP | 2015516832 A | 18-06-2015 |
| | | | RU | 2014143053 A | 20-05-2016 |
| | | | US | 2015063527 A1 | 05-03-2015 |
| | | | WO | 2013144812 A2 | 03-10-2013 |
| JP 7199920 | B2 | 06-01-2023 | JP | 7199920 B2 | 06-01-2023 |
| | | | JP | 2020074825 A | 21-05-2020 |